# EUROPÄISCHE PATENTSCHRIFT

(11) **EP 0 607 776 B1**
(45) Veröffentlichungstag und Bekanntmachung des Hinweises auf die Patenterteilung: **09.12.1998**
(21) Anmeldenummer: 94100016.8
(22) Anmeldetag: 03.01.1994
(51) Int. Cl.: A61K 31/42

(54) **Verwendung von Leflunomid zur Hemmung von Tumornekrosefaktor alpha**
Use of leflunomid for the inhibition of tumor necrosis factor alpha
Utilisation de leflunomid pour inhiber le facteur alpha de necrose tumorale

(30) Priorität: 08.01.1993 DE 4300280
(43) Veröffentlichungstag der Anmeldung: 27.07.1994
(73) Patentinhaber: HOECHST AKTIENGESELLSCHAFT, 65929 Frankfurt am Main (DE)
(72) Erfinder: Weithmann, Klaus Ulrich, Dr., D-65719 Hofheim (DE); Bartlett, Robert Ryder, Dr., D-64291 Darmstadt (DE)

(56) Entgegenhaltungen:
- EP-A- 0 013 376
- WO-A-91/17748
- WO-A-92/02822
- IMMUNOBIOLOGY Bd. 178, Nr. 1-2 , 1988 Seite 156 T. MATTAR ET AL. 'Effects of leflunomide (HWA 486) on mediators of the immune response'
- IMMUNOBIOLOGY Bd. 186, Nr. 1-2 , 1992 Seite 113 T. ZIELINSKI ET AL. 'Effects of leflunomide (HWA 486) on cell cycle distribution and expression of lymphyocyte activation markers'
- AGENTS AND ACTIONS Bd. 32, Nr. 1-2 , 1991 Seiten 10 - 21 R. BARTLETT ET AL. 'Leflunomide (HWA 486), a novel immunomodulating compound for the treatment of autoimmune disorders and reactions leading to transplantation rejection'
- CYTOKINE Bd. 5, Nr. 4 , Juli 1993 Seiten 298 - 304 T. SMITH-OLIVER ET AL. 'Elevated levels of tnfalpha in the joints of adjuvant arthritic rats'
- AGENTS AND ACTIONS Bd. 38, Nr. SPEC , 1993 Seiten C80 - C82 T. ZIELINSKI ET AL. 'Effects of leflunomide (HWA 486) on expression of lymphocyte activation markers'
- FEBS LETTERS Bd. 334, Nr. 2 , November 1993 Seiten 161 - 164 T. MATTAR ET AL. 'Inhibition of the epidermal growth factor receptor tyrosine kinase acitivty by leflunomide'

## Beschreibung

Leflunomid (s. Formel, N-(4-Trifluormethylphenyl)-5-methyl-isoxazol-4-carboxamid) ist als chemische Verbindung bereits bekannt (EP 0013376, EP 0217206, US 4351841, US 4965276).

Neben den bereits offenbarten antiphlogistischen Effekten bewirkt diese Substanz auch immunomodulatorische Wirkungen die sie zur Behandlung von Autoimmunerkrankungen und Transplantatabstossungsreaktionen befähigen. Es ist auch schon bekannt, daß ein Metabolit mit der Bezeichnung N-(4-Trifluormethylphenyl)-2-cyano-3-hydroxy-crotonsäureamid (s. Formel) für die heilenden Wirkungen von Leflunomid verantwortlich ist.

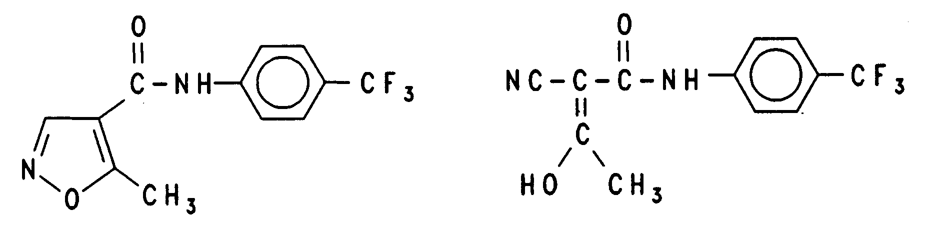

In Übereinstimmung mit dieser Erkenntnis können die oben zitierten pharmakologischen Wirkungen von Leflunomid auch durch Applikation dieses genannten Metaboliten erhalten werden (Bartlett et al, Agents and Actions, 32 (1991) 10-21).

Auch in Axton et al, J.Chem.Soc.Perkin Trans. 1 (1992) 2203ff ist beschrieben worden, daß nicht Leflunomid das aktive Prinzip darstellt, sondern daß dieser primäre Metabolit die biologischen Wirkungen entfaltet.

Sowohl in der Literatur (Bartlett et al, Agents and Actions, 32 (1991) 10-21) als auch in eigenen Experimenten konnte gezeigt werden, daß die im folgenden näher beschriebenen therapeutischen Wirkungen durch Applikation des Leflunomid-Metaboliten nicht erhalten werden können. Erfindungsgemäß wurde nämlich gefunden, daß Leflunomid einen starken inhibitorischen Einfluß auf die Synthese bzw. Freisetzung von Zytokinen aus menschlichen Blutzellen besitzt, wohingegen der Leflunomid-Metabolit diese vorteilhafte Wirkung nicht aufweist.

Unter den erfindungsgemäß angewandten experimentellen Bedingungen findet keine nennenswerte Metabolisierung des Leflunomid statt, und die inhibierende Wirkung ist ausschließlich der Substanz Leflunomid zuzuschreiben.

Bei den Zytokinen handelt es sich um eine Klasse verschieden-artiger, biologisch hochpotenter Peptide, deren Strukturen bereits bekannt sind. Es ist ebenfalls schon bekannt, daß sie endogen als Transmittersubstanzen induziert und synthetisiert werden.

Die Unterdrückung von Zytokinen im menschlichen oder tierischen Körper ist deshalb von großer medizinischer Bedeutung, weil überhöhte Spiegel dieser Zytokine zum Auftreten bzw. Ausbrechen zahlreicher Krankheiten führen können.

Zwar könnte zur Behandlung solcher Krankheiten auch ein Medikament eingesetzt werden, das die unerwünschte Wirkung des gegebenenfalls bereits vorhandenen Zytokines auf Organ-, Zell-, Gewebe- und Rezeptorsysteme des Körpers verhindert; es ist nun jedoch ein weiterer signifikanter Vorteil der vorliegenden Erfindung, daß durch Einsatz von Leflunomid bereits die Synthese bzw. Freisetzung des Zytokines verhindert wird, sodaß dieses gar nicht erst entsteht, und somit die Entstehung der Krankheit bereits in einer sehr frühen Phase verhindert werden kann.

Die vorliegende Erfindung betrifft die Verwendung von Leflunomid zur Herstellung eines Arzneimittels zur Vorbeugung und Behandlung von Krankheiten des menschlichen und tierischen Körpers, die durch das Zytokin mit der Bezeichnung Tumornekrosefaktor alpha (TNFalpha) ausgelöst werden, mit der Maßgabe, daß die Krankheiten rheumatische Arthritis und Krebserkrankungen ausgeschlossen sind.

TNFalpha und seine krankheitsverursachenden Wirkungen sind ausführlich in Ibelgaufts, Lexikon Zytokine, Medikon Verlag, München 1992, und in der dort zitierten Literatur beschrieben.

Auch z. B. aus WO 92/02822 kann entnommen werden, daß dieses Zytokin ein breites biologisches Aktivitätsspektrum besitzt.

TNFalpha wurde ursprünglich nach seiner ersten erkannten Wirkung, nämlich der Antitumorwirkung, benannt. Inzwischen ist auch beschrieben worden (Übersicht in Vilcek u. Lee, J.Biol.Chem. 266(1991)7313ff), daß TNFalpha breite pleiotrope Aktivitäten hat. Die Synthese von TNFalpha kann in zahlreichen Zellen erfolgen, ebenso besitzen viele Zellen spezifische Rezeptoren für dieses Zytokin, über die wiederum Wachstums- und Transkriptionsfaktoren, Akut-Phasenproteine und Zelladhäsionsmoleküle wie ELAM-1 und ICAM-1 (Bevilacqa et al, Science 243 (1989) 1160) induziert werden können.

TNFalpha besitzt eine potente Wirkung beim endotoxischen Schock (Tracey et al, Science 234 (1986) 470-474) und bei der Kachexie (Oliff et al, Cell, 50 (1987) 555-563. Ebenso wird der fatale Verlauf bei Malaria, bei Hirnhautentzündung (Waage et al, Lancet 1 (1987) 355-357), Lungenentzündung (Blanchard et al, Lymphokine Res 6 (1987) 1421), AIDS (Lahdevirta et al, Am.J.Med. 86 (1988) 289-291) sowie bei Krebspatienten (Balkwill et al, Lancet 2 (1987) 1229-1232) von TNFalpha beherrscht.

Es ist somit ersichtlich, daß insbesondere TNFalpha eine zentrale Stellung als Auslöser verschiedener Krankheiten und Krankheitssymptome einnimmt. Dabei handelt es sich überwiegend um schwerwiegende Krankheiten, deren Behandlung heute gar nicht oder nur unzureichend möglich ist. Auch deshalb kommt der aufgefundenen Wirkung von Leflunomid eine große Bedeutung zu.

Die vorliegende Erfindung betrifft ferner die Verwendung von Leflunomid zur Vorbeugung und Behandlung von Krankheiten wie septischer Schock, Kachexie, Malaria, Hirnhautentzündung, Lungenentzündung, Vergiftungen durch Endotoxine, Bindegewebeverdickungen, Lungenintoxikationen oder Lungenödemen (Tracy et al., Science, 234 (1986) Seite 314 ff.; Erroi et al., Agents Actions, 36 (1992) Seite 66 ff.; Scrip, 1713, Seite 15 (1992); Mustafa et al., J. Pediat., 115 (1989) Seite 1274 ff.).

Zur Behandlung dieser Krankheiten können insbesondere auch Arneimittelformen und galenische Zubereitungen von Leflunomid, die auf üblichem Wege hergestellt worden sind, Verwendung finden.

Geeignete feste oder flüssige galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit-oder Schmiermittel, Geschmacksstoffe, Süßungsmittel oder Lösungsvermittler, Verwendung finden. Als häufig verwendete Hilfsstoffe seien z. B. Magnesiumcarbonat, Titandioxid, Laktose, Mannit und andere Zucker, Talkum, Milcheiweiß, Gelatine, Stärke, Cellulose und ihre Derivate, tierische und pflanzliche Öle, Polyätthylenglykole und Lösungsmittel, wie etwa sterile Wasser und ein oder mehrwertige Alkohole, z. B. Glycerin, genannt.

In der Humanmedizin werden Dosiseinheiten mit 3 bis 5 mg, vorzugsweise 10, 25 oder 50 mg pro Patient(70 kg Körpergewicht) verabfolgt. Falls medizinisch erforderlich kann die Dosiseinheit bis auf 100, 200 oder 500 mg pro Patient gesteigert werden. Die Dosierung kann einmal täglich bis einmal wöchentlich, vorzugsweise bis zu drei oder viermal täglich erfolgen. Die Applikation kann oral, peritoneal, intravenös, intraartikulär oder transdermal auf übliche Weise erfolgen. Aus diesen Angaben lassen sich auch leicht die entsprechenden veterinärmedizinischen Applikationen berechnen.

Schließlich kann Leflunomid bei der Herstellung der vorgenannten galensichen Zubereitungsformen auch zusammen mit anderen geeigneten Wirkstoffen, beispielsweise Antiuricopathika, Thrombocytenaggregationshemmern, Analgetika und anderen steroidalen oder nicht-steroidalen Antiphlogistika, formuliert werden.

Experimentell konnten die Wirkungen von Leflunomid an einer isolierten Blutzellfraktion (mononukleare Zellen) nachgewiesen werden, die insbesondere keine nennenswerte Metabolisierung des Leflunomids zu seinem Metaboliten bewirkte.

### Beispiel 1

Die Anreicherung der mononuklearen Zellen aus frisch gewonnenem menschlichen Zitratblut erfolgte nach bekannten Standardverfahren (s. Tiku et al, J. Immunol. 136/10 (1986) 3677):

10 ml frisch hergestelltes menschliches Zitratblut wurden vorsichtig mit 15 ml Lymphoprep^{(R)} (Molter GmbH, Heidelberg) unterschichtet und dann bei 400xg 40 min bei 20° C zentrifugiert. Die sich in der Phasengrenze als weißer Ring abzeichnende Zellfraktion wurde mit Hilfe einer Spritze entnommen, 1:1 (v/v) mit PM-16-Puffer (Fa. Serva Feinbiochemica GmbH & Co KG, Heidelberg) verdünnt, und nochmals, wie oben, für 10 min zentrifugiert. Der Überstand wurde mit 10 ml RPMI 1640-Puffer (Gibco, Berlin), dem vorher 300 mg/l L-Glutamin zugesetzt worden waren, gewaschen. Die gewaschene Zellfraktion wurde in 1 ml RPMI 1640, dem vorher 300 mg /l L-Glutamin, 25 mmol/l HEPES (Gibco, Berlin), 0,1 g/ml Streptomyzin und 0,1 g/ml Penizyllin zugesetzt worden waren, aufgenommen. Mit Hilfe eines Zellzählers (Typ IT , Fa. Coulter Diagnostics, Krefeld) wurde die Zellsuspension, die aus ca. 90 % Lymphozyten und 10 % Monozyten besteht, auf etwa 5 Millionen Zellen/ml eingestellt. Die Zellviabilität wurde vor und nach den Inhibierungsexperimenten mit Hilfe der bekannten Laktatdehydrogenase-Methode überprüft. Eine Änderung der Viabilität wurde dabei nicht festgestellt.

Die Synthese und Freisetzung von zellulärem TNFalpha wurde induziert, indem zu 0,48 ml der oben beschriebenen Zellfraktion eine Lösung von 500 ng Lipopolysacharid (Salmonella abortus equi, Sigma GmbH, Deisenhofen) in 0,01 ml Dimethylsulfoxid/Wasser (1:10, v/v) gegeben wurde. Gleichzeitig wurde die Zellfraktion mit einer Lösung von Leflunomid oder Leflunomid-Metabolit in 0,01 ml Dimethylsulfoxid (jeweilige Endkonzentration siehe Tab. 1) versetzt, und das Gemisch wurde für 20 h bei 37° C in einem handelsüblichen Inkubator belassen. Nach dem Abkühlen auf 0° C wurden die Proben 1 min in einer Tischzentrifuge zentrifugiert, und jeweils 0,025-ml-Aliquote des Überstandes wurden mit Hilfe des "Sandwich-" Enzymimmuno-Testkits (Fa. Biermann GmbH, Bad Nauheim) nach Herstellervorschrift spezifisch auf ihren TNFalpha-Gehalt untersucht. Die Kontrollwerte wurden ohne Zusatz von Leflunomid oder seinem Metaboliten bestimmt und auf 100 % gesetzt. Insbesondere wurde durch entsprechende Vergleichs-messungen ein etwaiger Einfluß von Dimethylsulfoxid auf den TNFalpha-Spiegel ausgeschlossen.

Weiterhin wurden Aliquote des Leflunomid enthaltenden Testansatzes zeitabhängig entnommen und mit Hilfe der Hochdruckflüssigchromatographie (C-18-Säule 3,9x150 mm, Waters GmbH, Eschborn, Laufmittel: 600 ml Methanol/350 ml Wasser/ 50 ml Tetrahydrofuran/ 1 ml Phosphorsäure; Flußrate 0,7 ml/min bei 2000 pounds per square inch (Psi); Detektion im ultravioletten Bereich bei 273 nm) auf ihren Gehalt an Leflunomid oder Leflunomid-Metabolit überprüft. Es zeigte sich, daß Leflunomid unter den angewandten Bedingungen nur sehr langsam, mit einer Halbwertszeit von ca. 10 Stunden, zu seinem Metaboliten metabolisiert wird.

**TABELLE 1**

| Prüfsubstanz | Konzentration Versuche mmol/l | TNFalpha im Überstand % +/- Standard-Abweichung | Anzahl n = |
|---|---|---|---|
| Leflunomid-Metabolit | | | |
| | 0,1 | 105 | 2 |
| | 0,01 | 102 +/-12 | 3 |

| Leflunomid | | | |
|---|---|---|---|
| | 0,1 | 28 +/-5 | 3 |
| | 0,05 | 35 | 2 |
| | 0,01 | 100 +/-15 | 5 |
| ohne | 0 | 100 | |

Die Experimente in Tab. 1 zeigen, daß, der Leflunomid-Metabolit praktisch keinen Einfluß auf den TNFalpha-Spiegel bewirkt, während der TNFalpha-Spiegel nach Gabe von Leflunomid deutlich gesenkt wird.

### Beispiel 2

### Herstellung von N-(4-Trifluormethylphenyl)-5-methyl-isoxazol-4-carboxamid

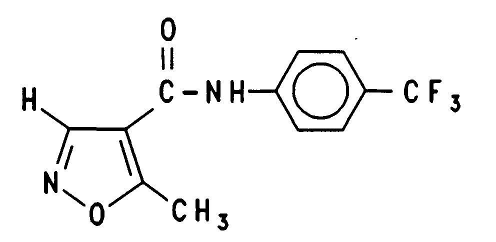

Eine Lösung von 0,05 Mol 4-Methylisoxazol-4-carbonsäurechlorid (7,3 g) in 20 ml Acetonitril werden tropfenweise bei Raumtemperatur in eine Lösung von 0,1 Mol 4-Trifluormethylanilin (16,1 g) in 150 ml Acetonitril gegeben. Nach 20 min Rühren wird das ausgefallene 4-Trifluormethylanilin-hydrochlorid abgesaugt, zweimal mit je 20 ml Acetonitril gewaschen und die vereingten Filtrate unter vermindertem Druck eingeengt. Ausbeute: 12,8 g weißes, kristallines N-(4-Trifluormethylphenyl)-5-methyl-isoxazol-4-carboxamid (Leflunomid).

### Beispiel 3

### Akute Toxizität nach intraperitonealer Verabreichung

Die akute Toxizität nach intraperitonealer Verabreichung der Testsubstanz wurde mit NMRI-Mäusen (20 bis 25 g) und SD-Ratten (120 bis 195 g) durchgeführt. Die Testsubstanz wurde in einer 1 %igen Natrium-Carboxymethylcellulose-Lösung suspendiert. Die verschiedenen Dosierungen der Testsubstanz wurden den Mäusen in einem Volumen von 10 ml/kg Körpergewicht und den Ratten in einem Volumen von 5 ml/kg Körpergewicht verabreicht. Pro Dosierung wurden 10 Tiere verwendet. Nach 3 Wochen wurde die akute Toxizität nach der Methode von Litchfield und Wilcoxon bestimmt. Die Ergebnisse sind in der Tabelle zusammengefaßt.

**Tabelle**

| | Leflunomid akute Toxizität intraperitoneal LD₅₀ (mg/kg) |
|---|---|
| NMRI-Maus | 185 (163 - 210) |
| SD-Ratte | 170 (153 - 189) |

## Patentansprüche

1. Verwendung von N-(4-Trifluormethylphenyl)-5-methyl-isoxazol-4-carboxamid zur Herstellung eines Arzneimittels zur Vorbeugung und Behandlung von Krankheiten, die durch Tumornekrosefaktor alpha ausgelöst werden, mit der Maßgabe, daß die Krankheiten rheumatische Arthritis und Krebserkrankungen ausgeschlossen sind.

2. Verwendung gemäß Anspruch 1, zur Vorbeugung und Behandlung von Krankheiten wie septischer Schock, endotoxischer Schock, Kachexie, Malaria, Hirnhautentzündung, Lungenentzündung, Vergiftungen durch Endotoxine, Bindegewebeverdickungen, Lungenintoxikationen oder Lungenödemen.

## Claims

1. The use of N-(4-trifluoromethylphenyl)-5-methylisoxazole-4-carboxamide for preparing a pharmaceutical for preventing and treating disorders which are triggered by tumor necrosis factor alpha, with the proviso that the disorders rheumatoid arthritis and cancer diseases are excluded.

2. The use as claimed in claim 1, for preventing and treating disorders such as septic shock, endotoxic shock, cachexia, malaria, meningitis, pneumonia, poisoning by endotoxins, connective tissue swellings, pulmonary intoxications or pulmonary edemas.

## Revendications

1. Utilisation du N-(4-trifluorométhylphényl)-5-méthylisoxazole-4-carboxamide pour la fabrication d'un médicament destiné à la prévention et au traitement de maladies qui sont déclenchées par le facteur de nécrose tumorale α, étant entendu que sont exclues les maladies polyarthrite rhumatoïde et maladies cancéreuses.

2. Utilisation selon la revendication 1, pour la prévention et le traitement de maladies telles que le choc septique, la cachexie, le paludisme, la méningite, la pneumonie, les empoisonnements par des endotoxines, les épaississements du tissu conjonctif, les intoxications pulmonaires ou les oedèmes pulmonaires.
